# EUROPEAN PATENT SPECIFICATION

(11) **EP 1 578 303 B1**
(45) Date of publication and mention of the grant of the patent: **14.04.2010**
(21) Application number: 03775658.2
(22) Date of filing: 03.12.2003
(51) Int. Cl.: A61C 17/16

(54) **SYSTEM FOR ENABLING THE USE OF PRODUCTS BEYOND THE END OF THE LIMITED TRIAL PERIOD**
SYSTEM ZUM ERMÖGLICHEN DER VERWENDUNG VON PRODUKTEN NACH ABLAUF VON DEREN TESTPERIODE
SYSTEME PERMETTANT D'UTILISER DES PRODUITS AU-DELA DE LA FIN D'UNE PERIODE D'ESSAI

(30) Priority: 18.12.2002 US 434610 P
(43) Date of publication of application: 28.09.2005
(73) Proprietor: Koninklijke Philips Electronics N.V., 5621 BA Eindhoven (NL)
(72) Inventor: BARMENTLO, Maarten, Briarcliff Manor, NY 10510-8001 (US); BAYEH, Daniel, Briarcliff Manor, NY 10510-8001 (US); PACE, John, W., Briarcliff Manor, NY 10510-8001 (US); GREZ, Joseph, W., Briarcliff Manor, NY 10510-8001 (US); BREWER, Gerald, K., Briarcliff Manor, NY 10510-8001 (US); TIURA, James, A., Briarcliff Manor, NY 10510-3001 (US)
(74) Representative: Schouten, Marcus Maria
(86) International application number: PCT/IB2003/005602
(87) International publication number: WO 2004/054466

(56) References cited:
- EP-A- 1 016 960
- EP-A- 1 150 196
- WO-A-01/93776
- US-A- 5 966 654
- US-A- 5 984 508

## Description

This invention relates generally to power toothbrushes and similar products, such as other personal appliances and more specifically concerns the enabling of those products for permanent (long-term) or other additional use after a trial period of use has concluded.

It is common practice in many industries to permit a potential customer a short time trial use of a product prior to a purchase decision. This practice has been successful, for example, for various software products and related services, where the use of the software is available for a limited time and then automatically terminates unless the user decides to purchase or license the product/service. It has also been used with other products, where actual use and satisfaction is a critical part of a purchasing decision, particularly where actual use convinces a potential customer to purchase when otherwise a purchase might not even be considered.

One example of a product where a period of trial use is helpful in making a purchase decision is a power toothbrush, which typically costs 20-30 times that of a manual toothbrush. Many potential customers for a power toothbrush will not consider such a purchase until they have experienced the benefits of the toothbrush during a trial period of actual use. The beneficial results of the power toothbrush then are apparent to them and they are then and only then willing to purchase. This is likely true for many other commercial products as well, such as other personal care products, including shavers. The concept of a trial use of a relatively expensive commercial product, such as a power toothbrush, is described in US 6 883 199, owned by the assignee of the present invention, and in US 5 966 654.

In such a case, the "trial" unit is basically a commercial unit, with full functional capability, but modified in some way to operate only for a specific limited time. A disadvantage of such a trial toothbrush or similar product, however, is that after the trial period is over, the apparatus is discarded.

It is desirable to maintain the basic concept of a trial unit, for products like power toothbrushes or other personal care appliances, which are fundamentally identical to a commercial unit, but which can be enabled by a specific action for permanent (long-term) or additional short-term or other use after the trial use time has been completed and the customer has arranged for payment.

Accordingly, the present invention, in one aspect, is a system for enabling limited time trial use products for additional preselected use, comprising: A power appliance which has been adapted for limited time trial use; and an enabling device provided to the user following authorization to enable the appliance for said additional use.

In another aspect, the invention is a system for enabling a limited time trial use product for additional preselected use, comprising; a power appliance that has been adapted for limited time trial use; and a communication element associated with the power appliance for receiving enabling message from an external source over a communication line and wherein the appliance includes a circuit for enabling the appliance for said preselected additional use in response to the communication element which has received an enabling message.

Another aspect of the present invention includes a system for enabling limited time trial use products for long-term use comprising: a power appliance that has been adapted for limited time trial use but otherwise has a full operational capability of a conventional version thereof, wherein the power appliance includes a portion thereof that enables the device for long-term use upon a selected one of 1) actuation and 2) de-actuation thereof.

A further aspect of the invention includes a system for enabling limited time trial use of products for long-term use, comprising: A power appliance that has been adapted for limited time trial use, the power appliance including an on/off switch operable by a user in a particular pattern, and wherein the power appliance includes a recognition circuit for recognizing a preselected pattern of operation of the on/off switch, the power appliance being enabled for long-term use following recognition of the select pattern.
Figures 1A, 1B and 1C show one embodiment of the present invention.
Figure 2 shows one alternative embodiment of the present invention.
Figure 3 shows another embodiment of the present invention.
Figure 4 shows another embodiment of the present invention.
Figure 5 is a simple flowchart showing the sequential steps of the enabling system of the present invention.

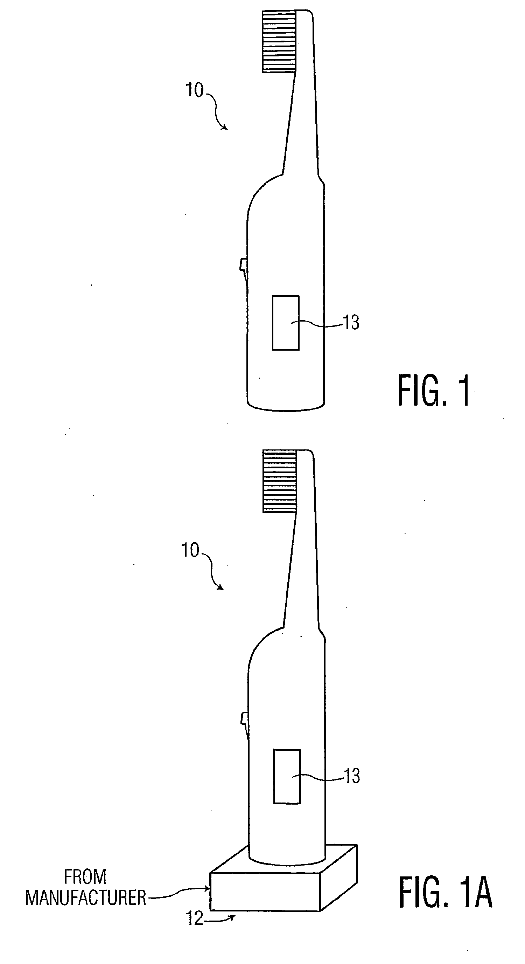

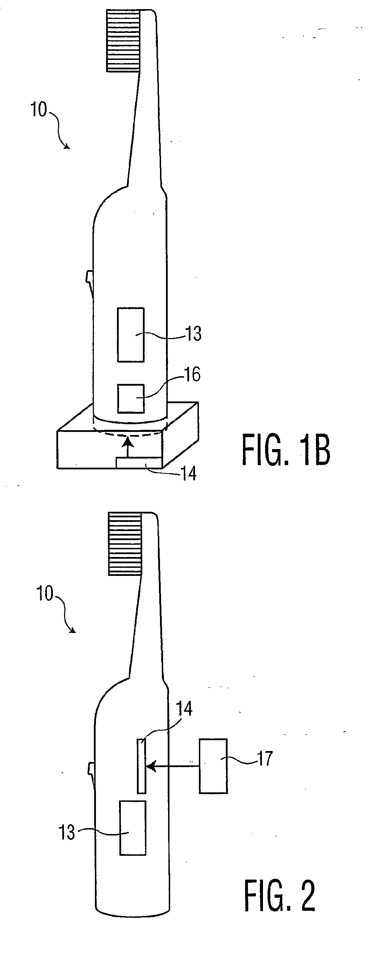

Figure 1 illustrates a first embodiment of the present invention in which, following a period of trial use, the user communicates with the manufacturer or a designated third party, such as a retail store or a dentist (in the case of a power toothbrush), to provide to the user an enabling element or device of some kind, following a payment by the user, to enable permanent operation (long-term, without a predetermined expiration) of the product. Figure 1 shows for illustration a power toothbrush 10 and an assembly or device 12 into which the toothbrush can be inserted, which will communicate with the product, *i.e.* the power toothbrush 10, to permanently enable the operation of the toothbrush, or other product, including specifically, other personal care appliances, such as shavers. This could include coded information, which is recognized by the microprocessor 13 in the toothbrush, which would then enable the toothbrush in accordance with programmed instructions.

Communication between device 12 and toothbrush 10 can be accomplished in various ways, such as magnetic action between a magnetic element 14 in device 12 and a corresponding magnetic element 16 in toothbrush 10. Alternatively, the communication could be optical, infrared (Ir) or RF (radio frequency). Other communication means could also be used. In all cases, the result is communication with the toothbrush (or other product), which results in the enablement of the toothbrush for permanent operation.

Alternatively to permanent operation, the enablement could be for an additional trial period or a period of use beyond a trial use, such as for one year, or the expected life of the brushhead. The cost of the enablement would depend upon the specific enablement requested. In addition, the enablement might be for specific functions only, such as a programmable timer or battery charge indicator or specific brushhead action.

In a variation of the arrangement of Figure 1, the device 12 is a charger assembly for the toothbrush, which would serve both to periodically charge the batteries in the toothbrush and also to provide communication with the toothbrush to enable permanent operation. This could include the various communication means discussed above, or it could involve a particular physical or electronic relationship with a given toothbrush such that the charger can only operate properly with the given toothbrush or other product. In such a case, the charger cannot be used to enable other toothbrushes.

In another variation of the embodiment of Figure 1, which is shown in Figure 2, an element or device is purchased by the user to enable the trial unit for long-term use (or other use as described above), a small, separate component 17 is integrated by the user into the toothbrush 10 through an opening 19 provided in the wall of the handle portion of the toothbrush. The additional element could, for instance, be used to complete an electrical circuit, which has opened at the end of the trial period, which results in enabling the device for permanent operation. Still further, the product (toothbrush) could be adapted to receive a keycard, which could be inserted into the product and then removed, the keycard either enabling communication with the product for thereafter enabling the product, or providing coded information recognizable by the product which results in its enablement. In such a case, the product would typically have a specific ID for communication.

The above embodiments, however, all include the basic feature that the user, by a purchase decision, receives an element or assembly which, when added or coupled to the product in a prescribed manner, results in the enabling of the product for permanent (long-term, i.e. no predetermined expiration) operation or for other specific operation described above.

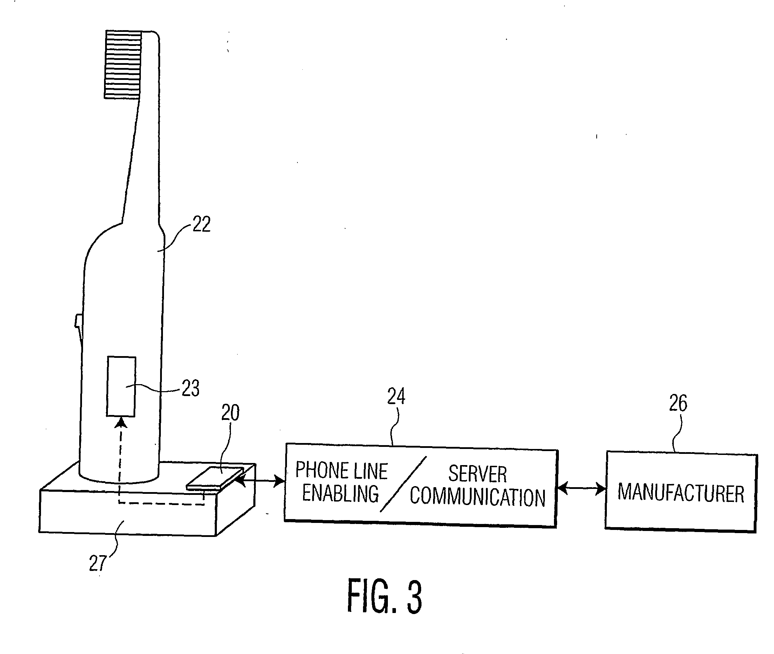

A second basic embodiment involves communication via telephone, cellular link or other communication line between the product and a component of the product and the manufacturer or other authorized third party for enablement of the product. In this arrangement, shown in Figure 3, a "smart" communication element 20 in a charger portion 21 of the toothbrush 22 or other personal care appliance (shaver, etc.) or other product is plugged into a phone line or other communication link 24. The communication line could, for instance, be an Internet access line. When payment for the permanent operation is made, the manufacturer or other authorized party 26 will activate the trial product in the possession of the user over the line, through the communication element 20 using an authorized communication protocol. The communication element 20 activates the appliance by communication with the microprocessor 23 or similar circuit in the appliance. In this embodiment, a communication protocol, instead of an actual element, is provided to the user, over a communication link.

This arrangement has certain advantages in simplicity, as well as reliability in ensuring that the trial product is enabled. The smart communication element 20 in the product 22 could be in a charging base for the toothbrush or in any other portion of the toothbrush. The user merely has to make the appropriate physical connection between element 20 and the communication line.

In addition to the enablement of operation of the product, a communication line connection could be used to perform remote diagnostics on the product to ensure/maintain proper operation of the product. It would also be used to ensure compliance in clinical situations for a toothbrush.

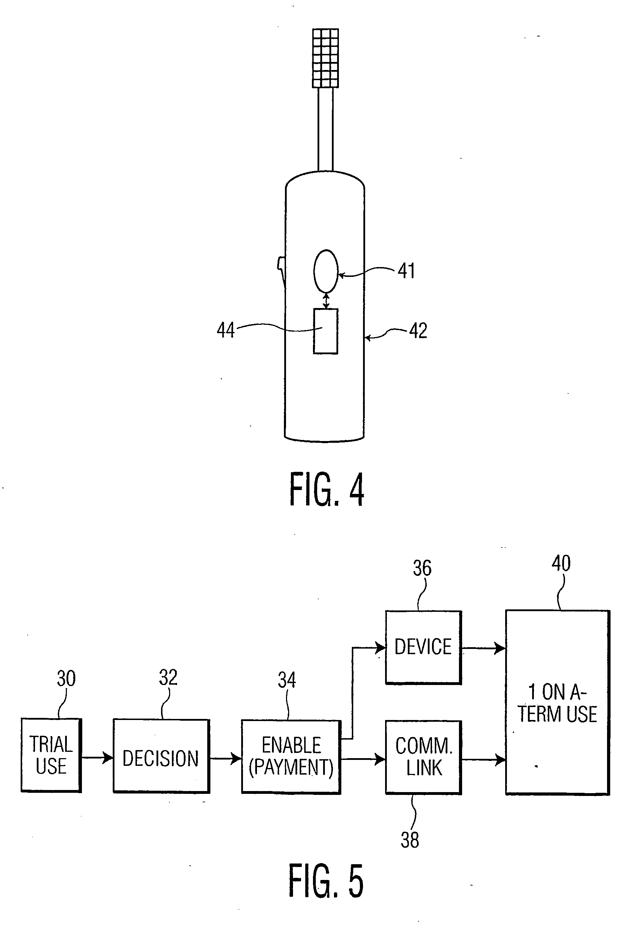

Figure 5 shows a simplified flowchart involving the first and second embodiments, in which a particular product, such as a power toothbrush, is sold in a trial use mode, as represented by block 30. During or following the trial use, a decision is made by the user to purchase a "permanent" version of the product, as shown in block 32. Other versions could also be purchased, including short-term usage or specific features (instead of the full function device). Contact is made with the manufacturer or other authorized party to enable the product, with accompanying payment, as shown in block 34. An element or device is provided to the user to enable conversion of the product to the desired operation status, as shown by block 36, or the product or accessory therefor, such as a charging unit, is connected via a communication link to the manufacturer or other authorized party, as shown in block 38. The product is now capable of permanent (long-term) use, *i.e.* basically identical in operation to the non-trial use products purchased through regular commercial channels, as represented by block 40, or other desired use/configuration. In both of these embodiments, the discarding of the trial use device is eliminated.

In another embodiment, shown in Figure 4, the user is provided with a particular "code" for operating the existing on/off switch 41 of the product (shown as a power toothbrush) 42, following payment. Operating the on/off switch 40 with a particular pattern over a selected period of time will result in enablement of the product for permanent (or selected other) use. The microprocessor 44 in the product is programmed to recognize the pattern from the on/off switch then to convert the product to permanent use. While this embodiment may in some cases experience difficulties if the user cannot properly carry out the specified on/off, it does have advantages in simplicity and lack of any additional expense in the enablement process.

In still another embodiment, a "physical" conversion of a portion of a circuit in the toothbrush or other product is used to produce permanent operation. For instance, when the product is in its trial mode, there may be a physical connection or link which prevents further operation of the product after a particular set time or number of uses of the product. Upon payment by the user, the product may be taken to a local authorized outlet, such as a dentist's office or other outlet, where the product may be passed through an enabling apparatus which either opens (blows) the existing disabling circuit link or produces a demagnetizing or other function in the product, in each case removing a blocking element or operating condition in the product which otherwise prevents permanent operation. This embodiment has the advantage of ensuring the enablement of the product without any action on the part of the user, other than a visit to a local outlet.

In still another embodiment, an initial period of enablement for a toothbrush (or other product using a replaceable workpiece) could be the expected life of the brushhead. Purchase of a new brushhead could then enable the product for the expected life of the new brushhead, i.e. six months.

In all of the above embodiments, there is a conversion operation of some kind involving an element of the product or communication with the microprocessor in the product, which is programmed to enable permanent (or other desired) operation of the product. Various means of communication with the product can be used. Conversion may occur by changing the operating state of existing elements of the product or in some cases may be accomplished by the actual physical elimination or addition of elements needed to produce such operation.

All of these embodiments, however, convert a power toothbrush or similar product, which has been initially set up for short-time trial use into a permanent operating device for normal use, in one example. The "enabled" product is identical in operation, function and appearance to a product which was initially purchased for full price. In other examples, the enablement could be for additional times of use (but not permanent) or could be for specific features only.

This system has the advantage of permitting a user to have a trial use of a product which otherwise might not have been purchased or even considered, without discard of the trial unit. A trial unit which has been permanently and completely enabled is identical in operation and function to a product conventionally purchased. Hence, there is an incentive for the user to enable the trial use unit, as opposed to purchasing a new product which is already configured for permanent use. The present invention thus provides an operation step or feature for a trial unit to enable the trial product without discarding of the trial unit. Trial use products thus become truly practical. The trial use product, as explained above, is advantageous, as it permits customers who are initially skeptical or even adverse to experience the advantages of the product and thus make an informed decision concerning purchase.

Although several embodiments have been disclosed for purposes of illustration, it should be understood that various changes, modifications and substitutions might be incorporated in the invention without departing from the scope of the invention, which is defined by the claims, which follow.

## Claims

1. A system for enabling limited time trial use products for additional preselected use, comprising:
a personal care appliance (10, 22, 42), which has been adapted for limited time trial use; and
an enabling device (12, 14, 41) for carrying out a conversion operation on the personal care appliance (10, 22, 42) in order to enable the appliance (10, 22, 42) for additional use, wherein the enabling device (12, 14, 41) is adapted to change an operation state of elements (13, 44) of the personal care appliance (10, 22, 42).

2. A system of claim 1, wherein the additional use is long-term use and includes all of the functions of a conventional product.

3. A system of claim 2, wherein the appliance is a power toothbrush.

4. A system of claim 2, wherein the enabling device is an element (17) which is positionable by the user permanently within the personal care appliance, the positioning of the device within the appliance resulting in said enabling of the personal care appliance.

5. A system of claim 2, wherein the enabling device is an element which is inserted temporarily into the device and then removed and wherein the personal care appliance includes a circuit which recognizes a code on the inserted element and enables the appliance for long-term use.

6. A system of claim 2, wherein the power toothbrush is brought into proximity to the enabling device, the power toothbrush and enabling device both having communication elements therein for communication therebetween to enable additional use of the personal care appliance.

7. A system of claim 6, wherein the power toothbrush nestles into the enabling device.

8. A system of claim 6, wherein the communication is optical.

9. A system of claim 6, wherein the communication is radio frequency (RF).

10. A system of claim 6, wherein the communication is magnetic.

11. A system of claim 6, wherein the communication is infrared.

12. A system of claim 2, wherein the enabling device is arranged to enable only one personal care appliance.

13. A system of claim 2, wherein the enabling device is capable of only one enabling operation.

14. A system for enabling a limited time trial use product for additional preselected use, comprising:
a personal care appliance (22) which has been adapted for a limited time trial use; and
a communication element (20) associated with the personal care appliance for receiving an enabling message from an external source (26) over a communication line (24) and
wherein the appliance includes a circuit or microprocessor (23) enabling the appliance for preselected additional use in response to the communication element receiving an enabling signal.

15. A system of claim 14, wherein the additional use is long-term use and includes all of the functions of a conventional product.

16. A system of claim 15, wherein the communication line is a telephone line.

17. A system of claim 15, wherein the power appliance is a power toothbrush.

18. A system of claim 15, wherein the communication line is an internet connection.

19. A system of claim 15, wherein the communication element is located in a charger portion of a personal care appliance.

20. A system for enabling limited time trial use products for long-term use, comprising:
a personal care appliance which has been adapted for limited time trial use but otherwise has a full operational capability of a conventional version thereof, wherein the personal care appliance includes a portion thereof which enables the device for long-term use upon a selected one of 1) actuation and 2) deactuation thereof.

21. A system of claim 20, wherein the personal care appliance is a power toothbrush.

22. A system for enabling limited time trial use of products for long-term use, comprising:
a personal care appliance which has been adapted for limited time trial use, the personal care appliance including an on/off switch operable by a user in particular patterns, and wherein the personal care appliance includes a recognition circuit for recognizing a preselected pattern of operation of the on/off switch, the personal care appliance being enabled for long-term use following recognition of the selected pattern.

23. A system of claim 22, wherein the personal care appliance is a power toothbrush.

## Patentansprüche

1. System zum Befähigen von Produkten mit zeitlich begrenzter Testverwendung zu weiterer vorgewählter Verwendung, wobei das System Folgendes umfasst:
ein Körperpflegegerät (10, 22, 42), das für eine zeitlich begrenzte Testverwendung vorgesehen wurde; und
eine Befähigungsvorrichtung 12, 14, 41) zum Durchführen eines Umwandlungsschritts an dem Körperpflegegerät (10, 22, 42), um das Gerät (10, 22, 42) zu einer zusätzlichen Verwendung (10, 22, 42) zu befähigen, wobei die Befähigungsvorrichtung (12, 14, 41) vorgesehen ist, um einen Betriebszustand von Elementen (13, 44) des Körperpflegegeräts (10, 22, 42) zu verändern.

2. System nach Anspruch 1, wobei die zusätzliche Verwendung eine langfristige Verwendung ist und alle Funktionen eines herkömmlichen Produkts beinhaltet.

3. System nach Anspruch 2, wobei das Gerät eine elektrische Zahnbürste ist.

4. System nach Anspruch 2, wobei die Befähigungsvorrichtung ein Element (17) ist, das durch den Benutzer dauerhaft in dem Körperpflegegerät angeordnet werden kann, wobei das Anordnen der Vorrichtung in dem Gerät die genannte Befähigung des Körperpflegegeräts zum Ergebnis hat.

5. System nach Anspruch 2, wobei die Befähigungsvorrichtung ein Element ist, das vorübergehend in die Vorrichtung eingesetzt wird und dann entfernt wird, und wobei das Körperpflegegerät eine Schaltung umfasst, die einen Code auf dem eingesetzten Element erkennt und das Gerät zur langfristigen Verwendung befähigt.

6. System nach Anspruch 2, wobei die elektrische Zahnbürste in die Nähe der Befähigungsvorrichtung gebracht wird, wobei sowohl die elektrische Zahnbürste als auch die Befähigungsvorrichtung mit Kommunikationselementen zur Kommunikation untereinander ausgestattet sind, um eine zusätzliche Verwendung des Körperpflegegeräts zu ermöglichen.

7. System nach Anspruch 6, wobei die elektrische Zahnbürste in die Befähigungsvorrichtung eingefügt wird.

8. System nach Anspruch 6, wobei die Kommunikation optisch erfolgt.

9. System nach Anspruch 6, wobei die Kommunikation per Funk erfolgt.

10. System nach Anspruch 6, wobei die Kommunikation magnetisch erfolgt.

11. System nach Anspruch 6, wobei die Kommunikation per Infrarot erfolgt.

12. System nach Anspruch 2, wobei die Befähigungsvorrichtung vorgesehen ist, um nur ein Körperpflegegerät zu befähigen.

13. System nach Anspruch 2, wobei die Befähigungsvorrichtung zu nur einer Befähigungsoperation in der Lage ist.

14. System zum Befähigen eines Produkts mit zeitlich begrenzter Testverwendung zu weiterer vorgewählter Verwendung, wobei das System Folgendes umfasst:
ein Körperpflegegerät (22), das für eine zeitlich begrenzte Testverwendung vorgesehen wurde; und
ein zu dem Körperpflegegerät gehörendes Kommunikationselement (20) zum Empfangen einer Befähigungsnachricht von einer externen Quelle (26) über eine Kommunikationsleitung (24), und wobei das Gerät eine Schaltung oder einen Mikroprozessor (23) umfasst, der das Gerät in Reaktion auf den Empfang eines Befähigungssignals durch das Kommunikationselement zur vorgewählten weiteren Verwendung befähigt.

15. System nach Anspruch 14, wobei die zusätzliche Verwendung eine langfristige Verwendung ist und alle Funktionen eines herkömmlichen Produkts beinhaltet.

16. System nach Anspruch 15, wobei die Kommunikationsleitung eine Telefonleitung ist.

17. System nach Anspruch 15, wobei das Körperpflegegerät eine elektrische Zahnbürste ist.

18. System nach Anspruch 15, wobei die Kommunikationsleitung eine Internetverbindung ist.

19. System nach Anspruch 15, wobei das Kommunikationselement in einem Ladeteil eines Körperpflegegeräts angeordnet ist.

20. System zum Befähigen von Produkten mit zeitlich begrenzter Testverwendung zu langfristiger Verwendung, wobei das System Folgendes umfasst:
ein Körperpflegegerät, das für eine zeitlich begrenzte Testverwendung vorgesehen wurde, aber ansonsten über die volle Funktionsfähigkeit einer herkömmlichen Ausführung hiervon verfügt, wobei das Körperpflegegerät einen Teil umfasst, das die Vorrichtung bei entweder 1) Aktivierung oder 2) Deaktivierung hiervon zur langfristigen Verwendung befähigt.

21. System nach Anspruch 20, wobei das Körperpflegegerät eine elektrische Zahnbürste ist.

22. System zum Befähigen von Produkten mit zeitlich begrenzter Testverwendung zu langfristiger Verwendung, wobei das System Folgendes umfasst:
ein Körperpflegegerät, das für eine zeitlich begrenzte Testverwendung vorgesehen wurde, wobei das Körperpflegegerät einen durch einen Benutzer in bestimmten Mustern betätigbaren Ein/Aus-Schalter umfasst, und wobei das Körperpflegegerät eine Erkennungsschaltung zum Erkennen eines vorgewählten Betätigungsmusters des Ein/Aus-Schalters umfasst, wobei das Körperpflegegerät im Anschluss an das Erkennen des ausgewählten Musters zu langfristiger Verwendung befähigt wird.

23. System nach Anspruch 22, wobei das Körperpflegegerät eine elektrische Zahnbürste ist.

## Revendications

1. Système de validation de l'utilisation d'essai de temps limité de produits pour une utilisation présélectionnée additionnelle, comprenant :
un ustensile d'hygiène corporelle (10, 22, 42) qui a été adapté à une utilisation d'essai de temps limité ; et
un dispositif de validation (12, 14, 41) pour effectuer une opération de conversion sur l'ustensile d'hygiène corporelle (10, 22, 42) afin de valider l'ustensile (10, 22, 42) pour une utilisation additionnelle, dans lequel le dispositif de validation (12, 14, 41) est adapté de manière à changer un état d'opération d'éléments (13, 44) de l'ustensile d'hygiène corporelle (10, 22, 42).

2. Système selon la revendication 1, dans lequel l'utilisation additionnelle est une utilisation à long terme et comprend toutes les fonctions d'un produit classique.

3. Système selon la revendication 2, dans lequel l'ustensile est une brosse à dents de puissance.

4. Système selon la revendication 2, dans lequel le dispositif de validation est un élément (17) qui est en permanence positionnable par l'utilisateur dans l'ustensile d'hygiène corporelle, le positionnement du dispositif dans l'ustensile ayant pour conséquence ladite validation de l'ustensile d'hygiène corporelle.

5. Système selon la revendication 2, dans lequel le dispositif de validation est un élément qui est inséré temporairement dans le dispositif et qui est ensuite enlevé, et dans lequel l'ustensile d'hygiène corporelle comprend un circuit qui reconnaît un code sur l'élément inséré et qui valide l'ustensile pour une utilisation à long terme.

6. Système selon la revendication 2, dans lequel la brosse à dents de puissance est mise à proximité du dispositif de validation, la brosse à dents de puissance et le dispositif de validation ayant des éléments de communication entre eux pour valider une utilisation additionnelle de l'ustensile d'hygiène corporelle.

7. Système selon la revendication 6, dans lequel la brosse à dents de puissance se niche dans le dispositif de validation.

8. Système selon la revendication 6, dans lequel la communication est optique.

9. Système selon la revendication 6, dans lequel la communication est une radiofréquence (RF).

10. Système selon la revendication 6, dans lequel la communication est magnétique.

11. Système selon la revendication 6, dans lequel la communication est infrarouge.

12. Système selon la revendication 2, dans lequel le dispositif de validation est agencé de manière à valider un seul ustensile d'hygiène corporelle.

13. Système selon la revendication 2, dans lequel le dispositif de validation est capable d'une seule opération de validation.

14. Système de validation d'une utilisation d'essai de temps limité d'un produit pour une utilisation présélectionnée additionnelle, comprenant :
un ustensile d'hygiène corporelle (22) qui a été adapté à une utilisation d'essai de temps limité ; et
un élément de communication (20) qui est associé à l'ustensile d'hygiène corporelle pour recevoir un message de validation en provenance d'une source externe (26) par le biais d'une ligne de communication (24), et dans lequel l'ustensile comprend un circuit ou microprocesseur (23) validant l'ustensile pour une utilisation présélectionnée additionnelle en réponse à l'élément de communication qui reçoit un signal de validation.

15. Système selon la revendication 14, dans lequel l'utilisation additionnelle est une utilisation à long terme et comprend toutes les fonctions d'un produit classique.

16. Système selon la revendication 15, dans lequel la ligne de communication est une ligne téléphonique.

17. Système selon la revendication 15, dans lequel l'ustensile de puissance est une brosse à dents de puissance.

18. Système selon la revendication 15, dans lequel la ligne de communication est une connexion internet.

19. Système selon la revendication 15, dans lequel l'élément de communication se situe dans une partie du chargeur d'un ustensile d'hygiène corporelle.

20. Système de validation d'une utilisation d'essai de temps limité de produits pour une utilisation à long terme, comprenant :
un ustensile d'hygiène corporelle qui a été adapté à une utilisation d'essai de temps limité mais qui présente autrement une pleine capacité opérationnelle d'une version classique de celui-ci, où l'ustensile d'hygiène corporelle en comprend une partie qui valide le dispositif pour une utilisation à long terme dans le cas de la sélection de 1) l'activation et de 2) la désactivation de celle-ci.

21. Système selon la revendication 20, dans lequel l'ustensile d'hygiène corporelle est une brosse à dents de puissance.

22. Système de validation d'une utilisation d'essai de temps limité de produits pour une utilisation à long terme, comprenant :
un ustensile d'hygiène corporelle qui a été adapté à une utilisation d'essai de temps limité, l'ustensile d'hygiène corporelle comprenant un commutateur marche/arrêt qui peut être actionné par un utilisateur dans des configurations particulières, et dans lequel l'ustensile d'hygiène corporelle comprend un circuit de reconnaissance pour reconnaître une configuration présélectionnée du fonctionnement du commutateur marche/arrêt, l'ustensile d'hygiène corporelle étant validé pour une utilisation à long terme après la reconnaissance de la configuration sélectionnée.

23. Système selon la revendication 22, dans lequel l'ustensile d'hygiène corporelle est une brosse à dents de puissance.
